# EUROPÄISCHE PATENTSCHRIFT

(11) **EP 1 454 141 B1**
(45) Veröffentlichungstag und Bekanntmachung des Hinweises auf die Patenterteilung: **21.05.2008**
(21) Anmeldenummer: 02785415.7
(22) Anmeldetag: 28.11.2002
(51) Int. Cl.: G01N 33/68

(54) **VERFAHREN UND VERWENDUNG EINES ANTIKÖRPERS ZUM NACHWEIS VON ALLERGIEN**
METHOD FOR DETECTING ALLERGIES AND USE OF AN ANTIBODY TO DETECT ALLERGIES
PROCEDE DE DETECTION D'ALLERGIES ET UTILISATION D'UN ANTICORPS POUR DETECTER DES ALLERGIES

(30) Priorität: 06.12.2001 DE 10160921
(43) Veröffentlichungstag der Anmeldung: 08.09.2004
(73) Patentinhaber: Eberhard-Karls-Universität Tübingen Universitätsklinikum, 72076 Tübingen (DE)
(72) Erfinder: BÜHRING, Hans-Jörg, 72076 Tübingen (DE)
(74) Vertreter: Otten, Hajo
(86) Internationale Anmeldenummer: PCT/EP2002/013437
(87) Internationale Veröffentlichungsnummer: WO 2003/048779

(56) Entgegenhaltungen:
- WO-A-00/72010
- RU-C- 2 008 687
- YAMAGUCHI MASAO ET AL: "Nonreleasing basophils convert to releasing basophils by culturing with IL-3." JOURNAL OF ALLERGY AND CLINICAL IMMUNOLOGY, Bd. 97, Nr. 6, 1996, Seiten 1279-1287, XP009007747 ISSN: 0091-6749
- BUEHRING HANS-JOERG ET AL: "The monoclonal antibody 97A6 defines a novel surface antigen expressed on human basophils and their multipotent and unipotent progenitors." BLOOD, Bd. 94, Nr. 7, 1. Oktober 1999 (1999-10-01), Seiten 2343-2356, XP002234798 ISSN: 0006-4971

## Beschreibung

Die Erfindung betrifft ein Verfahren zum Nachweis von Allergien.

Ein derartiges Verfahren ist aus der WO 00/72010 bekannt.

Allergien betreffen heutzutage weit über 20 % der Weltbevölkerung, und v.a. in den westlichen Ländern gibt es zunehmend mehr allergische Erkrankungen sowohl im Kindes- als auch im Erwachsenenalter.

Allergische Reaktionen können bspw. durch Pollen, Tierhaare und -schuppen, Nahrungs- und Arzneimittel, Hausstaubmilben, Insektengifte, etc. ausgelöst werden. Die ausgelösten allergischen Reaktionen äußern sich z.B. als Rhinitis, Hautausschlag, oder führen sogar zu einem allergischen Schock, meist in Abhängigkeit davon, mit welchen Körperregionen die Allergene in Kontakt geraten.

Insbesondere Wespen- oder Bienenstiche können bei Allergikern starke lokale Schwellungen oder schwere systemische - den gesamten Organismus erfassende - Störungen hervorrufen. Im Extremfall können allergische Reaktionen sogar bis zum Tod führen.

Allergische Reaktionen treten auf, wenn ein Individuum, das bereits IgE-Antikörper als Antwort auf ein harmloses Antigen produziert hat, anschließend wieder mit dem gleichen Allergen in Kontakt tritt. Diese Zweitexposition leitet ein Stadium der Überempfindlichkeitsreaktion ein.

Die Soforttyp-Allergien beruhen auf Überempfindlichkeitsreaktionen, die von IgE vermittelt werden. Die nach Kontakt mit einem Allergen in einer überschießenden Reaktion gebildeten IgE binden insbesondere an Mastzellen und an basophile Granulozyten (Basophile) an deren entsprechende IgE-Rezeptoren ("High-Affinity Rezeptoren", FceRI). Bei einem zweiten Kontakt mit dem Allergen bindet dieses die an die Rezeptoren der Zellen gebundenen IgE-Moleküle und vernetzt die IgE-Moleküle, wodurch eine Aktivierung dieser Zellen ausgelöst wird. Die gleiche Reaktion kann anstelle eines Allergens auch gezielt durch anti-IgE-Antikörper ausgelöst werden.

Als Folge werden von den aktivierten Zellen vorgefertigte chemische Mediatoren, wie bspw. Histamin, ausgeschüttet, die eine aktive Rolle in der Pathogenese von Überempfindlichkeitsreaktionen spielen.

Das Ausmaß der Histamin-Freisetzung aus Basophilen variiert unter den Patienten. Die -Basophilen einiger Patienten setzen sogar gar kein Histamin als Antwort auf anti-IgE frei; diese werden als sogenannte "Non-Responder" bezeichnet. Die genauen Schritte, die für diesen Freisetzungs-Mangel verantwortlich sind, sind bis heute ungeklärt. Gezeigt werden konnte aber, daß die IgE-Dichte auf den Basophilen-Oberflächen von Non-Respondern in etwa gleich ist wie die Ig-E-Dichte von Respondern, so daß der Freisetzungs-Mangel bspw. nicht auf eine geringere Anzahl an IgE-Rezeptoren zurückzuführen ist.

Yamaguchi M. et al., Nonreleasing Basophils Convert to Releasing Basophils by Culturing with IL-3, J. Allergy Clin. Immunol. 97: 1279-1287 (1996), beschrieben, -daß eine Histaminfreisetzung auch bei Basophilen von Non-Respondern induziert werden kann, wenn diese mit Interleukin IL-3 stimuliert wurden.

Die Diagnose von Hymenopteren-Allergien, also Allergien gegen Gifte von Hautflüglern wie bspw. Bienen oder Wespen, basiert hauptsächlich auf der Erfassung der Vorgeschichte, Hauttests und der Bestimmung von spezifischen IgE-Antikörpern. Um einen allergischen Status zu bestätigen, wird in ausgewählten Fällen die Allergen-induzierte Freisetzung von Histamin oder Leukotrien C4 gemessen.

Andere Tests beruhen auf durchflußzytometrischen Analysen, um entweder die Bindung von Fluoreszenz-markierten Allergenen an spezifische, zellgebundene IgE-Moleküle oder eine Allergen-induzierte Zelloberflächenexpression von basophilen Granula-Antigenen zu bestimmen.

So beschreiben bspw. Paris-Köhler A. et al., In vitro Diagnosis of Cypress Pollen Allergy by using Cytofluorimetric Analysis of Basophils (Basotest), J. Allergy Clin. Immunol. 105: 339-345 (2000), und Knol E. et al., Monitoring Human Basophil Activation via CD63 Monoclonal Antibody 435, J. Allergy Clin. Immunol. 88: 328-338 (1991), induzierte Zelloberflächenexpression des Granula-assoziierten Moleküls CD63 auf der Zelloberfläche von aktivierten Basophilen.

Irsch J. et al., The Frequency of Phospholipase A2 Binding of Basophilic Granulocytes does not decrease during Bee-venomspecific Immunotherapy, Allergy 54: 742-747 (1999), konnten die direkte Bindung von Phospholipase A₂ an Basophile von Patienten zeigen, die gegen Bienengift allergisch waren.

Die oben genannten Tests sind geeignet, um die Bindung von molekular definierten Allergenen zu analysieren, um die Soforttyp-Überempfindlichkeit zu detektieren und um auch möglicherweise den Erfolg einer Hyposensibilisierung bei allergischen Patienten zu untersuchen. Bei einer Hyposensibilisierung werden dem Patienten steigende Dosen eines Allergens injiziert, auf das diese empfindlich reagieren.

Die eingangs erwähnte WO 00/72010 beschreibt die Verwendung eines monoklonalen Antikörpers zum Nachweis von Allergien. Dabei wird die Tatsache ausgenutzt, daß bei der Aktivierung von Basophilen durch ein Allergen bestimmte Oberflächenstrukturen dieser Zellen verstärkt exprimiert werden. Der in dieser Druckschrift beschriebene Antikörper bindet an derartige, verstärkt exprimierte Oberflächenstrukturen, und dient damit auch der Quantifizierung von aktivierten und nicht-aktivierten Basophilen. Weiterhin wird ein Verfahren zum Nachweis von Allergien mit diesem Antikörper beschrieben.

Nachteilig bei diesem Verfahren und bei den oben genannten Tests ist, daß sie bei sogenannten Non-Respondern nicht eingesetzt werden können. Wie weiter oben dargestellt, findet bei Non-Respondern keine Freisetzung von Histamin und keine Hochregulierung von Oberflächenstrukturen statt, obwohl die betreffenden Patienten allergisch gegen ein bestimmtes Allergen sein können. Non-Responder können zwar positiv in Hauttests sein oder spezifische IgE-Antikörper produzieren, jedoch können Non-Responder durch ein Verfahren, mit dem aktivierte und nichtaktivierte Basophile, bzw. die Hochregulierung bestimmter Oberflächenstrukturen erfaßt werden, nicht als Allergiker identifiziert werden.

So bleibt nach einem Test mit den üblichen Verfahren stets eine bestimmte Ungewißheit, ob die als Non-Responder identifizierten Patienten-Proben auch tatsächlich Nicht-Allergiker sind.

Die RU-C-2008 687 offenbart ein Verfahren zur Diagnose verspäteter allergischer Reaktionen durch IL-3-Gabe zur Blutprobe, wobei die Konzentration von Basophilen gemessen wird. Bei einer Verminderung der Basophilen-Konzentration von 10 % oder mehr wird eine verspätete allergische Reaktion diagnostiziert.

Vor diesem Hintergrund ist es Aufgabe der vorliegenden Erfindung, ein Verfahren bereitzustellen, mit dem auch die Non-Responder unter den Allergikern erfolgreich diagnostiziert werden können.

Diese Aufgabe wird erfindungsgemäß durch ein Verfahren zum Nachweis von Allergien bei Individuen gelöst, welche in Allergie-Tests als Non-Responder identifiziert wurden, mit den Schritten:
- Vorinkubieren einer Blutprobe eines Non-Responders mit Interleukin IL-3,
- anschließend Inkubieren dieser Blutprobe mit einem Allergen,
- Inkubieren dieser Blutprobe mit einem Antikörper, der an diejenigen Oberflächenstrukturen von Basophilen und/oder Mastzellen und/oder von Vorläuferzellen von Basophilen und/oder Mastzellen bindet, an die der Antikörper mit der Bezeichnung 97A6 binden kann, der von Hybridomzellen produziert und freigesetzt wird, die am 12.2.1997 unter der Nummer DSM ACC 2297 bei der Deutschen Sammlung von Mikroorganismen und Zellkulturen GmbH, DSMZ, gemäß dem Budapester Vertrag hinterlegt worden sind,
- Quantifizierung der an die Basophilen und/oder Mastzellen und/oder Vorläuferzellen der Basophilen und/oder Mastzellen gebundenen Antikörper.

Die der Erfindung zugrundeliegende Aufgabe wird auf diese Weise vollkommen gelöst.

Die Erfinder der vorliegenden Anmeldung haben nämlich erkannt, daß es möglich ist, nach einer Behandlung von Non-Respondern mit Interleukin IL-3 und anschließender Inkubation mit einem bestimmten Allergen die Oberflächenstruktur von Basophilen und/oder Mastzellen und/oder Vorläuferzellen von Basophilen und/oder Mastzellen hochzuregulieren, an die der Antikörper 97A6 binden kann. Interleukin IL-3 induziert zwar selbst eine Hochregulierung dieser Oberflächenstruktur, die zusätzliche Hochregulierung durch das Allergen ist jedoch um mindestens den Faktor zwei höher und daher meßtechnisch erfaßbar.

Die Allergiker unter den Non-Responder können von den herkömmlichen Tests, d.h. Tests, bei denen der Status von Basophilen bestimmt wird, nicht erfaßt werden, da bei ihnen nach Allergenkontakt weder Histamin freigesetzt, noch eine bestimmte Oberflächenstruktur hochreguliert wird, um einen allergischen Status zu bestätigen.

Die Erfinder zeigten, daß bei den Allergikern unter den Non-Respondern durch das erfindungsgemäße Verfahren eine bestimmte Oberflächenstruktur hochreguliert werden konnte. Durch die anschließende Inkubation mit einem Antikörper, der die hochregulierte Oberflächenstruktur erkennt, konnten daher die Allergiker unter den Non-Respondern identifiziert und der Allergietyp eindeutig zugeordnet werden.

Bei nicht-allergischen Non-Respondern wurde nach einer Inkubation mit Interleukin IL-3 die Oberflächenstruktur durch ein Allergen nicht spezifisch hochreguliert, so daß diese auch als tatsächliche Nicht-Allergiker bestätigt werden konnten.

Interleukine sind Zytokine, die hauptsächlich durch Leukozyten nach physiologischen oder nicht-physiologischen Stimuli abgegeben werden, und Lymphozyten sowie ihre Vorläuferzellen aus dem Knochenmark, aber auch andere Blutzellen im Hinblick auf deren Proliferation, Differenzierung und Zell-Zell-Interaktionen beeinflussen.

Interleukin IL-3 wird hauptsächlich von Antigen-aktivierten T-Lymphozyten, aber auch bspw. von Endothelzellen, Mastzellen, Monozyten usw., gebildet. Es hat starken Einfluß auf Wachstum und Differenzierung hämatopoetischer Stammzellen und fördert die Freisetzung von Histamin und Leukotrien C4 aus basophilen Granulozyten.

Durch Vergleichsmessungen zu Interleukin IL-3 lassen sich weitere Agentien bestimmen, die die erfindungsgemäße Wirkung haben. Dazu zählen Agentien, die Zellen bspw. durch eine proliferierende und/oder stimulierende und/oder aktivierende Wirkung beeinflussen.

Die Erfinder konnten zeigen, daß durch Inkubation von Blutproben mit Interleukin IL-3 Oberflächenstrukturen hochreguliert wurden, an die der Antikörper 97A6 bindet. Die Oberflächenstruktur ist Phosphodiesterase/Nukleotidpyrophosphatase-3 (PDNP3), die auch als E-NPP3 oder PD-Ibeta bezeichnet wird. Die Sequenz ist veröffentlicht von Jin-Hua et al. in Genomics 45: 412-415 (1997).

In einer Ausführungsform ist bevorzugt, wenn eine Interleukin IL-3-Menge von 1 bis 20 ng/ml, vorzugsweise von ca. 10 ng/ml, verwendet wird.

Bei einer bevorzugten Ausführungsform wird die Blutprobe mit Interleukin IL-3 zwischen einer Stunde und 96 Stunden, vorzugsweise über Nacht, also für ca. 24 Stunden, inkubiert.

Die Erfinder konnten zeigen, daß bei 24 h der Stimulationsindex optimal war, um auch bei Respondern den Test durchzuführen.

Weiterhin ist bevorzugt, wenn als Antikörper ein monoklonaler Antikörper, insbesondere der Antikörper 97A6 verwendet wird, der von Hybridomzellen produziert und freigesetzt wird, die am 12.2.1997 unter der Nummer DSM ACC 2297 bei der Deutschen Sammlung von Mikroorganismen und Zellkulturen GmbH, DSMZ, gemäß dem Budapester Vertrag hinterlegt worden sind. Die Aufbewahrungsdauer wurde entsprechend verlängert.

Dieser Antikörper ist in der klinischen Praxis bereits erprobt und wird von der Firma Beckman Coulter unter der Bestellnummer IM3575 vertrieben.

Dieser Antikörper erkennt spezifisch ein Epitop der Oberflächenstruktur Phosphodiesterase/Nukleotidpyrophosphatase-3 (PDNP3), die bei Allergikern Allergen-spezifisch hochreguliert wird.

Darüber hinaus kann auch ein Antikörper verwendet werden, der an ein anderes Epitop von PDNP3 als der Antikörper 97A6 bindet.

Dadurch kann eine Verstärkung des Nachweissignals erzielt werden, da dann mehr Antikörper an eine Zelle binden können.

In einer Ausführungsform ist bevorzugt, wenn ein Antikörper verwendet wird, der mit einem Marker, insbesondere mit einem Fluoreszenz-Marker verbunden ist.

Das Antigen kann dadurch mit einer hohen Sensitivität nachgewiesen werden, weshalb dann nur geringe Mengen des Antikörpers zum Nachweis eingesetzt werden müssen.

Der Nachweis gebundener Antikörper erfolgt dann bspw. durch übliche immunologische Nachweisverfahren, bspw. ELISA (Enzyme-linked-Immunosorbent Assay) oder durch eine FACS-Analyse. Die Verwendung dieser Nachweisverfahren bietet den Vorteil einer hochspezifischen, schnellen und sensitiven Analyse von Zellen.

Erfindungsgemäß wird das Verfahren zum Nachweis von Allergien bei Proben eingesetzt, die in Allergie-Tests als Non-Responder identifiziert wurden.

Der Einsatz dieses Verfahrens erst nach einem ersten Allergen-Test ohne Inkubation mit Interleukin IL-3, hat den Vorteil, daß es kostengünstig speziell nur bei den Proben eingesetzt werden kann, die sich in dem ersten Test als Non-Responder erwiesen haben. Durch den Anschluß des erfindungsgemäßen Verfahrens ist gewährleistet, daß auch die Non-Responder unter den Allergikern erkannt werden können.

In weiteren Ausführungsformen ist es bevorzugt, wenn IL-3 zur Durchführung des Nachweises von Allergien bei den Proben verwendet wird, die in einem ersten Test mit dem Allergen einen Non-Responder-Status aufwiesen.

Bei weiteren Ausführungsformen wird ein Antikörper, der an diejenigen Oberflächenstrukturen von Basophilen und/oder Mastzellen und/oder Vorläuferzellen von Basophilen und/oder Mastzellen bindet, an die der Antikörper mit der Bezeichnung 97A6 binden kann, zum Nachweis von Allergien von Non-Respondern verwendet.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Vorteile ergeben sich aus dem folgenden Ausführungsbeispiel.

### Beispiel

Blutzellen von Non-Respondern (Allergiker und Normalpersonen) und von Respondern wurden für unterschiedliche Zeitintervalle (eine Stunde, 24 Stunden, 48 Stunden, 96 Stunden) mit 10 ng/ml Interleukin IL-3 bei einer Temperatur von 37°C stimuliert (3 ml peripheres Vollblut in 10 ml Vollmedium plus 10 ng/ml Interleukin IL-3).

Die Inkubation erfolgte in 25 cm² Gewebekulturflachen bei 37°C und bei 5 % CO₂. Die Expression von E-NPP3 wurde zu den Zeitpunkten Null Stunden (vor Inkubation), eine Stunde, ein Tag, zwei Tage, drei Tage (bzw. vier Tage), sechs Tage nach Interleukin IL-3-Inkubation gemessen.

Anschließend wurden die Proben jeweils mit seriellen Verdünnungen an Bienen- und Wespengift oder PBS (Phosphat-buffered Saline) plus Calcium (Negativkontrolle) oder Anti-IgE (Positivkontrolle) für 15 Minuten bei 37° inkubiert.

Die Reaktion wurde mit 20 mmol EDTA-Lösung abgestoppt und die Zellen in 50 *µ*l FACS-Puffer (0,1% NaN₃ + 0,1 % BSA, in "Hank's balanced salt solution") resuspendiert. Die Zellen wurden dann mit 10 *µ*l 97A6-PE Antikörper (1 µg/ml Endkonzentration) 15 min lang bei Raumtemperatur inkubiert.

Anschließend wurden 2 ml Erythrozytenlyse-Reagens- (Versalyse, Immunotech) zugegeben und für 10 min bei Raumtemperatur inkubiert. Daraufhin wurden 2 ml FACS-Puffer zugegeben und die Probe bei 1.200 u/min für 7 min zentrifugiert. Der Überstand wurde verworfen, dem Pellet wurden 4 ml FACS-Puffer zugegeben und die Probe wurde ein weiteres Mal bei 1.200 u/min für 7 min zentrifugiert. Nach Verwerfung des Überstandes wurde das die Zellen enthaltende Pellet in 150 *µ*l FACS-Puffer aufgenommen und auf Eis aufbewahrt.

Danach wurden die Zellen in der Durchflußzytometrie (FACScalibur) analysiert. Die Veränderung der E-NPP3-Expression wurde unter Berechnung des Stimulationsindex (SI) und des Prozentsatzes der stimulierten Basophilen bestimmt. Der Stimulationsindex wird berechnet aus: mittlere Fluoreszenzintensität von Allergen- oder Anti-IgE-stimulierten 97A6⁺-Zellen / mittlere Fluoreszenzintensität von PBS-stimulierten 97A6⁺-Zellen.

### Ergebnis:

Die Ergebnisse zeigten, daß die optimale Stimulationsdauer für die Bestimmung der Allergen-spezifischen, E-NPP3-Hochregulierung 24 Stunden (optimaler Stimulationsindex) war.

Bei den untersuchten allergischen und nicht-allergischen Non-Respondern konnte auch nach IL-3-Stimulierung keine wesentlich verbesserte anti-IgE-induzierte E-NPP3-Hochregulierung festgestellt werden.

Überraschenderweise konnte jedoch bei den zur Verfügung stehenden allergischen Non-Respondern eine Allergen-spezifische E-NPP3-Hochregulierung nach IL-3-Stimulierung beobachtet werden. In beiden Fällen konnte der Allergietyp (jedes Mal Wespe und nicht Biene) bestätigt werden, der nach der Anamnese im Hauttest und Bestimmung von spezifischem IgE erhoben wurde.

## Patentansprüche

1. Verfahren zum Nachweis von Allergien bei Individuen die in Allergie-Tests als Non-Responder identifiziert wurden, mit den Schritten:
- Vorinkubieren einer Blutprobe eines Non-Responders mit Interleukin IL-3,
- anschließend Inkubieren dieser Blutprobe mit einem Allergen,
- Inkubieren dieser Blutprobe mit einem Antikörper, der an diejenigen Oberflächenstrukturen von Basophilen und/oder Mastzellen und/oder Vorläuferzellen von Basophilen und/oder Mastzellen bindet, an die der Antikörper 97A6 binden kann, der von Hybridomzellen produziert und freigesetzt wird, die am 12.02.1997 unter der Nummer DSM ACC 2297 bei der Deutschen Sammlung von Mikroorganismen und Zellkulturen GmbH, DSMZ, gemäß dem Budapester Vertrag hinterlegt worden sind, und
- Quantifizierung der an die Basophilen und/oder Mastzellen und/oder Vorläuferzellen der Basophilen und/oder Mastzellen gebundenen Antikörper.

2. Verfahren nach Anspruch 1, **dadurch gekennzeichnet, daß** eine Interleukin IL-3-Menge von 1 bis 20 ng/ml, vorzugsweise von ca. 10 ng/ml verwendet wird.

3. Verfahren nach Anspruch 1 oder 2, **dadurch gekennzeichnet, daß** die Blutprobe mit Interleukin IL-3 zwischen einer Stunde und 96 Stunden, vorzugsweise für ca. 24 Stunden, inkubiert wird.

4. Verfahren nach einem der Ansprüche 1 bis 3, **dadurch gekennzeichnet, daß** als Antikörper ein monoklonaler Antikörper verwendet wird.

5. Verfahren nach einem der Ansprüche 1 bis 4, **dadurch gekennzeichnet, daß** als Antikörper der Antikörper 97A6 verwendet wird, der von Hybridomzellen produziert und freigesetzt wird, die am 12.2.1997 unter der Nummer DSM ACC 2297 bei der Deutschen Sammlung von Mikroorganismen und Zellkulturen GmbH, DSMZ, gemäß dem Budapester Vertrag hinterlegt worden sind.

6. Verfahren nach einem der Ansprüche 1 bis 5, **dadurch gekennzeichnet, daß** ein Antikörper, der mit einem Marker, insbesondere einem Fluoreszenz-Marker, verbunden ist, verwendet wird.

7. Verwendung eines Antikörpers, der an diejenigen Oberflächenstrukturen von Basophilen und/oder Mastzellen und/oder Vorläuferzellen von Basophilen und/oder Mastzellen bindet, an die der Antikörper mit der Bezeichnung 97A6 binden kann, zur Durchführung eines Verfahrens nach einem der Ansprüche 1 bis 6, zum Nachweis von Allergien bei Individuen, die in Allergie-Tests als Non-Responder identifiziert wurden.

## Claims

1. A method for detecting allergies in individuals, which have been identified in allergy tests as being nonresponders, which comprises the steps of:
- incubating a blood sample of a nonresponder with interleukin IL-3,
- incubating this blood sample with an allergen,
- incubating this blood sample with an antibody which binds to the surface structures on basophils and/or mast cells and/or on precursor cells of basophils and/or mast cells to which the antibody 97A6 can bind, which antibody is produced and released by hybridoma cells which were deposited in the Deutsche Sammlung von Mikroorganismen und Zellkulturen [German collection of microorganisms and cell cultures] GmbH, DSMZ, under number DSM ACC 2297, in accordance with the Budapest Treaty, on 12.2.1997, and
- quantifying the antibodies which are bound to the basophils and/or mast cells and/or precursor cells of the basophils and/or mast cells.

2. The method according to claim 1, **characterized in that** a quantity of interleukin IL-3 of from 1 to 20 ng/ml, preferably of approx. 10 ng/ml, is used.

3. The method according to claim 1 or 2, **characterized in that** the blood sample is incubated with interleukin IL-3 for between one hour and 96 hours, preferably for approx. 24 hours.

4. The method according to any of claims 1 to 3, **characterized in that** the antibody employed is a monoclonal antibody.

5. The method according to any of claims 1 to 4, **characterized in that** the antibody employed is the antibody 97A6, which is produced and released by hybridoma cells which were deposited in the Deutsche Sammlung von Mikroorganismen und Zellkulturen [German collection of microorganisms and cell cultures] GmbH, DSMZ, under number DSM ACC 2297, in accordance with the Budapest treaty, on 12.2. 1997.

6. The method according to any of claims 1 to 5, **characterized in that** use is made of an antibody which is linked to a label, in particular a fluorescent label.

7. Use of an antibody, which binds to the surface structures on basophils and/or mast cells and/or precursor cells of basophils and/or mast cells to which the antibody designated 97A6 can bind, for detecting allergies in individuals which have been identified in allergy tests as being nonresponders, for implementing a method according to one of claims 1 to 6.

## Revendications

1. Procédé pour la détection d'allergies chez des individus qui, lors de tests d'allergie, ont été identifiés comme étant des non-répondeurs, comprenant les étapes consistant à :
- pré-incuber un échantillon sanguin d'un non-répondeur avec l'interleukine IL-3,
- consécutivement, incuber cet échantillon sanguin avec un allergène,
- incuber cet échantillon sanguin avec un anticorps qui se lie sur les structures superficielles des basophiles et/ou des mastocytes et/ou des cellules précurseur des basophiles et/ou des mastocytes, sur lesquelles peut se lier l'anticorps 97A6, qui est produit et libéré par les cellules hybridomes qui ont été déposées le 12.02.1997 sous le numéro DSM ACC 2297 auprès de la Deutsche Sammlung von Mikroorganismen und Zellkulturen GmbH, DSMZ, conformément au Traité de Budapest, et
- quantification des anticorps liés aux basophiles et/ou aux mastocytes et/ou aux cellules précurseur dés basophiles et/ou des mastocytes.

2. Procédé selon la revendication 1, **caractérisé en ce que** l'on utilise une quantité d'interleukine IL-3 de 1 à 20 ng/ml, de préférence d'environ 10 ng/ml.

3. Procédé selon la revendication 1 ou 2,
**caractérisé en ce que** l'échantillon sanguin est incubé avec l'interleukine IL-3 entre 1 heure et 96 heures, de préférence pendant environ 24 heures.

4. Procédé selon l'une des revendications 1 à 3, **caractérisé en ce qu'**en tant qu'anticorps, on utilise un anticorps monoclonal.

5. Procédé selon l'une des revendications 1 à 4, **caractérisé en ce qu'**en tant qu'anticorps, on utilise l'anticorps 97A6 qui est produit et libéré par les cellules hybridomes qui ont été déposées le 12.02.1997 sous le numéro DSM ACC 2297 auprès de la Deutsche Sammlung von Mikroorganismen und Zellkulturen GmbH, DSMZ, conformément au Traité de Budapest.

6. Procédé selon l'une des revendications 1 à 5, **caractérisé en ce que** l'on utilise un anticorps qui est lié à un marqueur, en particulier un marqueur de fluorescence.

7. Utilisation d'un anticorps qui se lie sur les structures superficielles des basophiles et/ou des mastocytes et/ou les cellules de précurseur de basophiles et/ou les mastocytes, sur lesquelles peut se lier l'anticorps portant la désignation 97A6 pour la réalisation d'un procédé selon l'une des revendications 1 à 6, pour détecter des allergies chez les individus qui ont été identifiés comme étant des non-répondeurs lors de tests d'allergie.
